# EUROPÄISCHE PATENTSCHRIFT

(11) **EP 1 579 876 B1**
(45) Veröffentlichungstag und Bekanntmachung des Hinweises auf die Patenterteilung: **05.10.2011**
(21) Anmeldenummer: 05006066.4
(22) Anmeldetag: 19.03.2005
(51) Int. Cl.: A61L 31/04, A61L 31/14

(54) **Zusammensetzung zur Adhäsionsprophylaxe**
Composition for post-operative adhesion prevention
Composition pour la préventions d'adhérences post-chirurgicales

(30) Priorität: 22.03.2004 DE 102004014633
(43) Veröffentlichungstag der Anmeldung: 28.09.2005
(73) Patentinhaber: Aesculap AG, 78532 Tuttlingen (DE)
(72) Erfinder: Odermatt, Erich K., CH-8200 Schaffhausen (CH); Härtel, Claudia, D-78573 Wurmlingen (DE); Weis, Christine, D-78532 Tuttlingen (DE); Holl, Christian, D-89079 Ulm (DE)
(74) Vertreter: Patentanwälte Ruff, Wilhelm, Beier, Dauster & Partner

(56) Entgegenhaltungen:
- WO-A-02/09789
- US-A- 6 133 325

## Beschreibung

Die Erfindung betrifft eine Zusammensetzung zur Adhäsionsprophylaxe für die postoperative Verhinderung von Verwachsungen im Körper, die Verwendung der Zusammensetzung, einen Kit enthaltend mindestens zwei im wesentlichen getrennte Behältnisse, die die Komponenten der Zusammensetzung enthalten, sowie die Bereitstellung der Zusammensetzung zur Verwendung für die Adhäsionsprophylaxe.

Bei jedem chirurgischen Eingriff am menschlichen Körper besteht aufgrund unzureichender enzymatischer fibrinolytischer Aktivität das Risiko von dauerhaften, festen Adhäsionen zwischen Organen und bzw. Gewebeteilen, die zu erheblichen funktionellen Störungen an Organen, zu einer verzögerten Wundheilung und allgemein zu anhaltenden Schmerzen führen können. Diese Adhäsionsprobleme können bei einer Vielzahl von chirurgischen Eingriffen auftreten wie beispielsweise bei Laparoskopien im Bereich der Gynäkologie, bei denen entstandene Verwachsungen häufig Unterleibsschmerzen und Infertilität bedingen können, in der Herzchirurgie beim Einsatz bzw. Austausch von künstlichen Herzklappen bzw. der Anlage von aortokoronaren Bypässen sowie in der Nephrologie in Form von peritonealen Adhäsionen bei der Peritonealdialyse.

Diese Adhäsionen führen zu längeren medizinischen Behandlungszeiten, zu chronischen Schmerzen des Patienten und zu erhöhten Kosten durch Behebung der Adhäsionen.

Es wurde bisher versucht, durch Verbesserung operationstechnischer Maßnahmen und der chirurgischen Techniken allgemein die Gefahr von Verwachsungen und die damit verbundenen Komplikationen zum reduzieren. Allein diese Maßnahmen reichen jedoch nicht aus, so dass weitere additive Maßnahmen zur Prophylaxe postoperativer Adhäsionen notwendig sind.

Es wurden bisher eine Vielzahl an Substanzen zur Adhäsionsprophylaxe vorgeschlagen, angefangen von Glaskörperflüssigkeit von Kalbsaugen oder Olivenöl bis hin zu Antikoagulantien und Fibrinolytika, die jedoch alle nur einen geringen Rückgang der Adhäsionen mit sich brachten. Die lokale Anwendung von Antibiotika hat sogar überwiegend zu einer Verstärkung der Adhäsionsbildung geführt. Es wurden weiterhin Produkte zur Adhäsionsprophylaxe aus Polyvinylalkoholen eingesetzt (vgl. WO 02/09789), die jedoch den Nachteil aufweisen, dass die Haftung auf dem Gewebe ungenügend war und damit die Membran nicht am Applikationsort verblieb.

Der Erfindung stellt sich daher die Aufgabe, eine Zusammensetzung zum Einsatz bei chirurgischen Eingriffen zur Verhinderung von postoperativen Verwachsungen im Körper zur Verfügung zu stellen, die bei einer Vielzahl von chirurgischen Eingriffen einfach applizierbar, dauerhaft elastisch, gut haftend sowie einfach und kostengünstig herzustellen ist und in der Praxis auch an schwer zugänglichen Regionen während der Operation leicht handhabbar ist.

Diese Aufgabe wird gelöst durch eine Zusammensetzung zur Adhäsionsprophylaxe für die postoperative Verhinderung von Verwachsungen im Körper aus mindestens drei, insbesondere drei, Komponenten, wobei mindestens zwei Komponenten derart miteinander vernetzbar sind, dass die Zusammensetzung nach der Vernetzung eine homogene gelartige Masse ist, umfassend mindestens die folgenden Komponenten: (a) eine wässrige, insbesondere viskose, Lösung einer Carboxymethylcellulose (CMC), (b) eine wässrige Lösung mindestens eines Vernetzungsmittels, insbesondere eines Gelbildners und (c) eine wässrige Lösung eines Polyvinylalkohols (PVA). Die einzelnen Komponenten der Zusammensetzung liegen in der Regel in einem solchen Zustand vor, dass sie noch nicht miteinander reagieren können. Die Komponenten können aber auch in einem Zustand vorliegen, in dem sie bereits miteinander reagiert haben. Die Vernetzung ist dabei so gesteuert, dass das Vernetzungsprodukt noch applizierbar ist. Mit Vorteil kann die erfindungsgemäße Zusammensetzung als Gel, Spray aber auch als Folie, Lösung oder Schaum vorgesehen sein.

Alle in der Zusammensetzung enthaltenen Komponenten sind ausgezeichnet bioverträglich, verursachen keine unerwünschten Nebenwirkungen und können nach dem enzymatischen Abbau oder der Resorption über die Niere zum größten Teil ausgeschieden werden. Die Abbau- bzw. die Ausscheidungsgeschwindigkeit sind durch den Anteil der verschiedenen Komponenten sowie durch Zusatzstoffe einstellbar.

Es ist möglich, dass die Komponente (a) und/oder die Komponente (c) mit der Komponente (b) vernetzbar ist. In einer besonderen Ausführungsform sind die Komponenten (a) und (b) miteinander vernetzbar und die Komponenten (b) und (c) liegen in einer gemeinsamen, von der Komponente (a) separierten, Lösung vor. Es ist auch möglich, die Komponenten (b) und (c) miteinander zu vernetzen. In einer weiteren Ausführungsform enthält die Komponente (b) zwei Vernetzungsmittel (b1) und (b2) und sowohl die Komponente (a) wie auch die Komponente (c) sind durch die Komponente (b) (b1+b2) vernetzbar, und die 3 Komponenten liegen jeweils separat vor. Alternativ kann die Komponente (b1) gemeinsam mit Komponente (a) und Komponente (b2) mit Komponente (c) vorliegen, wobei (a) mit (b2) und (c) mit (b1) vernetzbar ist.

Mit Vorteil ist die Vernetzungszeit der erfindungsgemäßen Zusammensetzung je nach Art der Anwendung individuell einstellbar. Die Regulierung der Vernetzungszeit erfolgt insbesondere durch die Art und die Mengenverhältnisse der mindestens zwei miteinander vernetzbaren Komponenten.

Bei einer besonderen Ausführungsform sind die einzelnen Komponenten und deren Mengenverhältnisse untereinander so ausgewählt, dass die Zusammensetzung eine Vernetzungszeit von 5 Sekunden bis 10 Minuten, vorzugsweise von 10 Sekunden bis 5 Minuten und insbesondere ca. 30 bis 60 Sekunden beträgt. Weiterhin kann die Vernetzungszeit durch Zugabe von Inhibitionssubstanzen, vorzugsweise Komplexbildnern, insbesondere Chelatkomplexe wie EDTA, oder Zitronensäure, verzögert werden. Die ausgewählte Vernetzungszeit ist jeweils von der Applikationsart und vom Ort der Applikation, insbesondere der Beschaffenheit der Gewebeoberfläche, auf die die Zusammensetzung appliziert wird, abhängig. Außerdem spielt die Viskosität der Zusammensetzung für die Auswahl der Vernetzungszeit eine besondere Rolle, so dass bei hochviskosen Zusammensetzungen eine längere Vernetzungszeit und bei niederviskosen Zusammensetzungen eine kürzere Vernetzungszeit einstellbar ist.

In einer Ausführungsform ist die Vernetzungszeit der Zusammensetzung durch die Konzentration der einzelnen vernetzbaren Komponenten einstellbar. So kann durch eine hohe Konzentration mindestens einer der beiden miteinander vernetzbaren Komponenten eine kurze Vernetzungszeit erzielt werden. Es ist auch möglich, durch eine niedrige Konzentration der beiden miteinander vernetzbaren Komponenten eine relativ lange Vernetzungszeit einzustellen. Weiterhin kann die Vernetzungszeit durch eine höhere, insbesondere im Bereich von ca. 37 °C liegende, Temperatur erheblich verkürzt werden, wohingegen durch Vorkühlen bzw. Verarbeitung bei niedrigeren Temperaturen, insbesondere bei Raumtemperatur von ca. 22 °C, die Vernetzungszeit verlängert werden.

Es ist weiterhin möglich, die Vernetzungszeit der Zusammensetzung durch den Grad der Vermischung der einzelnen Komponenten einzustellen. Je intensiver die Vermischung der einzelnen zunächst separat voneinander vorliegenden Komponenten erfolgt, desto stärker kann die Vernetzungszeit verkürzt werden. Andererseits ist es auch möglich durch eine geringe Durchmischung der einzelnen miteinander vernetzbaren Komponenten die Vernetzungszeit auszudehnen.

Hinsichtlich der Applikation ist es einerseits möglich, die Komponenten vor der Vernetzung zu mischen und anschließend zu applizieren, wobei die Vernetzungszeit im Falle der Durchmischung vor der Applikation entsprechend länger gewählt ist als im Falle der separaten Applikation der einzelnen Komponenten vor der Vernetzung. In einer bevorzugten Ausführungsform ist jede Komponente vor der Vernetzung separat verstreichbar oder versprühbar. Insbesondere kann eine Komponente zunächst auf die Applikationsstelle vorgestrichen werden und anschließend mit der zweiten Komponente versehen, vorzugsweise besprüht, werden, um die Vernetzung auszulösen.

Mit Vorteil ist die erfindungsgemäße Zusammensetzung vor der Vernetzung auf dem Körpergewebe oder Organ, insbesondere direkt aus einer Breitschlitzdüse eines statischen oder dynamischen Mischers einer Applikationsvorrichtung, verstreichbar und weist vorteilhafterweise eine Viskosität von 0,1 bis 150 Pas, vorzugsweise ca. 10 Pas bei ca. 37 °C, auf.

In einer anderen Ausführungsform ist die erfindungsgemäße Zusammensetzung vor der Vernetzung vorzugsweise durch Luft, Sauerstoff, Stickstoff oder Kohlendioxid versprühbar. Zum Versprühen weist die Zusammensetzung vorzugsweise eine Viskosität von 1,5 bis 2 Pas bei 22 °C auf.

Im Fall einer durch Versprühen applizierten Zusammensetzung wird diese vorteilhafterweise bei einem Druck von 0,5 x 10⁵ Pa bis 5 x 10⁵ Pa (0,5 bis 5 bar), vorzugsweise bei 1 x 10⁵ Pa bis 2 x 10⁵ Pa (1 bis 2 bar), versprüht, um das traumatisierte sensible Körpergewebe nicht durch einen zu hohen Druck zu beschädigen. Eine Düsenöffnung von 0,3 bis 2 mm, vorzugsweise 0,5 bis 1,5 mm, insbesondere ca. 1 bis 1,2 mm, Durchmesser hat sich bei der Sprühapplikation als vorteilhaft erwiesen.

In einer besonderen Ausführungsform enthält die Zusammensetzung mindestens ein Mittel zur Erhöhung der Viskosität, vorzugsweise ein Verdickungs- oder ein Geliermittel, insbesondere aus der Gruppe umfassend Xanthan, Pullulan, Curdlan, Alginate, Agar-Agar, Carrageen, Furcelleran, Pektin, Johannesbrotkernmehl, Guarkernmehl, Tarakernmehl, Gummiarabikum, Traganth, Karaya, Cellulose und Stärke. Vorteilhafterweise beträgt die Menge an Verdickungs- oder Geliermittel 0,01 bis 10 Gew% der Zusammensetzung. Es ist möglich, nur einer Komponente ein Mittel zur Erhöhung der Viskosität zuzusetzen. Vorteilhafterweise wird beiden Komponenten zur Einstellung einer annähernd gleichen Viskosität jeweils eine entsprechende Menge an Verdickungsmittel zugesetzt, um insbesondere im Fall der Sprühapplikation zwei Komponenten mit gleicher Viskosität durch Mischen während des Versprühens applizieren zu können.

In einer Ausführungsform enthält die Komponente (a) 0,1 bis 5 Gew%, vorzugsweise 2 bis 4 Gew% und insbesondere ca. 3 Gew% Carboxymethylcellulose (CMC). Bei einer flüssigen oder gelartigen Auftragung durch eine Flachschlitzdüse hat sich eine 3 Gew% CMC-Komponente als vorteilhaft erwiesen, wohingegen bei einer Sprühapplikation vorzugsweise lediglich 2 Gew% CMC in der Komponente (a) enthalten sind.

Mit Vorteil weist die Komponente (a) eine Viskosität (37 °C) von 0,5 Pas bis 5 Pas, insbesondere von ca. 2,5 Pas bei einer 3 Gew% CMC-Lösung, auf.

Der Substitutionsgrad an Carboxymethylgruppen der Komponente (a) beträgt in einer Ausführungsform 0,2 bis 2,8 %, bevorzugt 0,4 bis 1,2 %, insbesondere ca. 0,7 %.

In einer Ausführungsform enthält die Komponente (c) 1 bis 20 Gew%, vorzugsweise 5 bis 15 Gew% und insbesondere ca. 10 Gew% Polyvinylalkohol (PVA). Der Anteil an PVA ist abhängig vom Anteil an CMC in Komponente (a), da der PVA als Mittel zur Reduzierung der Sprödigkeit der Carboxymethylcellulose dient und entsprechend im Falle einer höheren CMC-Konzentration vorteilhafterweise ebenfalls in einer höheren Menge in Komponente (c) enthalten ist.

Bei einer bevorzugten Ausführungsform weist die Komponente (c) eine Viskosität (37 °C) von 0,1 bis 100 Pas, vorzugsweise 0,1 bis 50 Pas, insbesondere 0,5 bis 20 Pas, und insbesondere ca. 1,4 Pas bei einer 10 Gew% PVA-Lösung, auf. Bei einer 15 Gew% PVA-Lösung beträgt die Viskosität vorzugsweise 14,5 Pas. In vorteilhafter Ausführung weist der Polyvinylalkohol ein Molekulargewicht von 20 000 bis 400 000, vorzugsweise 100 000 bis 300 000, und insbesondere ca. 200 000 auf. Durch das Molekulargewicht des PVA's kann die Ausscheidungsgeschwindigkeit reguliert werden. Je höher das Molekulargewicht des Polyvinylalkohols, desto länger benötigt der Körper, um die Produkte über die Niere auszuscheiden.

In einer Ausführungsform beträgt das Volumenverhältnis von Komponente (a) zu Komponente (b/c) 1 : 9 bis 9 : 1. In einer bevorzugten Ausführungsform beträgt das Volumenverhältnis der Komponente (a) zu Komponente (b/c) 1 : 1. Das Volumenverhältnis 1 : 1 ist insbesondere für die Bereitstellung der Komponenten in einer Doppelkammerspritze von Vorteil, da beide Kammern damit den gleichen Durchmesser und den gleichen Ausstoß pro Zeiteinheit haben. Außerdem sind die Viskositäten der beiden Komponenten in den beiden Kammern der Doppelkammerspritze vorzugsweise gleich.

In einer bevorzugten Ausführungsform ist das Volumenverhältnis der Komponente (c) zu Komponente (b) in einer gemeinsamen Lösung (c/b) ca. 20 : 1. Je nach dem Volumenverhältnis der beiden Komponenten kann die Konzentration der Komponenten (b) und (c) variiert werden.

Bevorzugte Konzentrationen der Komponenten sind Tabelle 1 bis 3 sowie den Beispielen zu entnehmen.

In einer bevorzugten Ausführungsform enthält die Komponente (b) 0,001 bis 2 Gew% und insbesondere 0,1 bis 0,5 Gew% Vernetzungsmittel.

In vorteilhafter Weise handelt es sich bei dem Vernetzungsmittel der Komponente (b) zur Vernetzung mit der Komponente (a) um eine Lösung mehrwertiger Kationen, insbesondere mehrwertiger Metallionen, vorzugsweise Aluminium-, Eisen(III)- und/oder Kupfer(II)ionen. Dabei kann es sich um mindestens eines aus der Gruppe umfassend Aluminiumacetat, Aluminiumchlorid, Aluminiumsulfat, Eisen(III)chlorid, Eisen(III)sulfat, Eisen(III)carbonat, Kupfer(II)chlorid und Kupfer(II)sulfat handeln. Besonders bevorzugt handelt es sich um Eisen(III)chlorid (FeCl₃).

In einer anderen Ausführungsform handelt es sich bei dem Vernetzungsmittel der Komponente (b) zur Vernetzung mit der Komponente (a) um eine Lösung mehrwertiger organischer Kationen. Dabei handelt es sich vorzugsweise um Polyaminosäuren, insbesondere um Poly-Lysine. Es ist auch möglich, dass es sich bei der Komponente (b) um nichtmetallische Kationen, insbesondere um kationische Naturstoffe, handelt. In einer Ausführungsform handelt es sich um mindestens eines aus der Gruppe Polylysin, Diethylaminoethyldextran (DEAE-Dextran), Polyethylenimin (PEI), Polydimethylaminoethylmethacrylat (PDMAEMA), Polyamidoamin Dentrimere und kationische Lipide, insbesondere Lipopolyamine und Lipopoly-L-lysine.

Als Komponente (b) zur Vernetzung der Komponente (c) (PVA) eignen sich mehrwertige organische Säuren, insbesondere Zitronensäure. Hierbei ist es bevorzugt, dass nicht gleichzeitig mehrwertige Kationen, insbesondere mehrwertige Metallionen, als Vernetzer für die Komponente (a) (CMC) vorliegen, da mehrwertige organische Säuren, insbesondere Zitronensäure die mehrwertigen Metallionen maskieren können und so sich die beiderseitigen Vernetzer gegenseitig beeinflussen können. Die mehrwertigen Säuren können auch in Form ihrer wasserlöslichen Salze vorliegen. Geeignet hierzu sind beispielsweise Silbersalze wie Silberzitrat bzw. Silberdihydrogenzitrat. Die Silberionen können dann gleichzeitig als antibakterieller Zusatz wirken.

In einer Ausführungsform erfolgt die Vernetzung der Komponenten untereinander durch Komplexbildung. Hierbei ist eine koordinative Bindung wie ionische, van der Waal- oder H-Brücken-Bindung bevorzugt.

Mit Vorteil weist die erfindungsgemäße Zusammensetzung einen Wassergehalt von mindestens 80 %, vorzugsweise 80 bis 90 % auf.

In einer weiteren Ausführungsform kann die Zusammensetzung neben den vernetzbaren Komponenten auch einen antibakteriellen Zusatz, insbesondere Silbernanopartikel, oder einen antiphlogistischen Zusatz, insbesondere Allantoin, enthalten. Vorteilhafterweise werden diese Wundheilungsagenzien und/oder Entzündungshemmer zur Verbesserung der Wundheilung der Zusammensetzung beigesetzt, wobei das wasserlösliche Allantoin vorzugsweise in einer Konzentration von 0,01 bis 0,2 Gew% der Komponente (a) und/oder der Komponente (c) zugesetzt werden kann.

Mit Vorteil ist die erfindungsgemäße Zusammensetzung in vollständig vernetztem Zustand immer noch flexibel und kann dadurch im Gegensatz zu einer PVA-freien vernetzten CMC-Zusammensetzung alle Körper- bzw. Organbewegungen mitmachen, ohne sich aufgrund mangelnder Elastizität vom Gewebe abzulösen oder durch die Bewegungen Risse zu erhalten. Die erfindungsgemäße Zusammensetzung ist vorteilhafterweise sehr elastisch und verfügt über eine ausreichende Dehnbarkeit, um auch auf sich stark ausdehnenden Organen bzw. Geweben dauerhaft und sicher zu haften.

Die Zusammensetzung ist vorzugsweise in komplett abgefülltem Zustand, insbesondere durch Dampf, e-beam und/oder γ-Strahlung, sterilisierbar.

Die Zusammensetzung aus mindestens drei, insbesondere drei, Komponenten, wobei mindestens zwei Komponenten derart miteinander vernetzbar sind, dass die Zusammensetzung nach der Vernetzung eine homogene gelartige Masse ist, umfassend mindestens die folgenden Komponenten: (a), eine wässrige, insbesondere viskose, Lösung einer Carboxymethylcellulose (CMC), (b), eine wässrige Lösung mindestens eines Vernetzungsmittels, insbesondere eines Gelbildners und (c), eine wässrige Lösung eines Polyvinylalkohols (PVA), kann weiterhin zur Adhäsionsprophylaxe für die postoperative Verhinderung von Verwachsungen im Körper verwendet werden, wobei die vernetzbaren Komponenten während der Applikation auf dem Körpergewebe miteinander vermischt werden.

Vorteilhafterweise werden die einzelnen vernetzbaren Komponenten gleichzeitig auf das Gewebe bzw. das Organ aufgebracht und mit dem Aufbringen ausreichend vermischt, um die Vernetzungsreaktion zu ermöglichen.

Weiterhin kann die Zusammensetzung zur Adhäsionsprophylaxe verwendet werden, wobei die vernetzbaren Komponenten kurz vor der Applikation miteinander vermischt werden. Die vernetzbaren Komponenten können vorteilhafterweise in einem statischen oder dynamischen Mischer gemischt und sofort nach dem Vermischen durch eine einzelne Austrittsöffnung appliziert werden.

Weiterhin kann die Zusammensetzung zur Adhäsionsprophylaxe verwendet werden, wobei die zu vernetzenden Komponenten nacheinander auf die Applikationsstelle aufgetragen werden und erst auf der Applikationsstelle miteinander vermischt werden. Dabei kann die Applikation mindestens einer Komponente durch Verstreichen und/oder die Applikation mindestens einer weiteren Komponente durch Aufsprühen auf die Applikationsstelle erfolgen. Es ist möglich, die Komponenten (a) und (c) oder (a/c) auf die Applikationsstelle in Form von Gelen aufzustreichen und anschließend die Komponente (b) auf die bestrichene Applikationsstelle zu sprühen, um die Vernetzung zu initiieren.

Bei einer anderen Ausführungsform ist es auch möglich, die Komponenten a, b und c in bereits vernetzter Form vorzulegen. Bei dieser Ausführungsform ist es vorteilhaft, die Konzentrationen und Mengenverhältnisse der einzelnen Komponenten so einzustellen, dass sich eine Viskosität des bereits vernetzten Gels im Bereich von 3 bis 50 Pas, insbesondere 3 bis 20 Pas, ergibt. Auch ein solches bereits vernetztes Gel weist gute antiadhäsive Eigenschaften auf. Bei dieser Ausführungsform reichen Applikatoren mit nur einer Kammer aus.

Weitherin kann die Zusammensetzung zur Adhäsionsprophylaxe verwendet werden, wobei die Komponenten nach dem Mischen durch Versprühen vorzugsweise mit einem Druck von 0,5 x 10⁵ Pa bis maximal 2 x 10⁵ Pa (0,5 bis maximal 2 bar), auf das Gewebe appliziert werden.

Außerdem kann die Zusammensetzung zur Adhäsionsprophylaxe verwendet werden, wobei die einzelnen zu vernetzenden Komponenten separat oder in Kombination mit weiteren Komponenten durch Versprühen appliziert werden. Vorteilhafterweise werden die Komponenten in der Reihenfolge (a) + (b/c) oder (a/c) + (b) appliziert.

Die Erfindung umfasst weiterhin einen Kit, der mindestens zwei, vorzugsweise zwei, im wesentlichen getrennte Behältnisse enthält, wobei die Behältnisse mindestens eine der Komponenten (a), (b) und (c) der Zusammensetzung zur Adhäsionsprophylaxe enthalten. Bei den Behältnissen handelt es sich vorteilhafterweise um die Kammern einer Mehrkammerspritze.

In einer Ausführungsform besteht der Kit aus zwei Behältnissen, wobei das eine die Komponente (a) und das andere die Komponenten (b/c) enthält. Bei dieser Aufteilung der Komponenten (a), (b) und (c) werden vorteilhafterweise gleiche Volumen aus den zwei Behältnissen miteinander vermischt.

In einer anderen Ausführungsform besteht der Kit aus zwei Behältnissen, wobei das eine die Komponenten (a/c) und das andere lediglich die Komponente (b) enthält. Auch bei dieser Aufteilung werden vorteilhafterweise gleiche Volumina aus den zwei Behältnissen miteinander vermischt.

Bei einer besonderen Ausführungsform enthält der Kit eine Vorrichtung zum Mischen der vernetzbaren Komponenten. Dabei handelt es sich vorteilhafterweise um einen statischen oder dynamischen Mischer.

Bei einer weiteren Ausführungsform ist der Kit für den minimal invasiven, insbesondere laparoskopischen, chirurgischen Einsatz ausgelegt.

Die Erfindung umfasst außerdem die Bereitstellung der Zusammensetzung aus mindestens drei, insbesondere drei, Komponenten zur Verwendung zur Adhäsionsprophylaxe für die postoperative Verhinderung von Verwachsungen im Körper, zur Behandlung von Leckagen im thorakalen und abdominalen Bereich sowie für die Auffüllung von AVMs (arteriovacuösen malformations) und Aneurismen.

Weitere Merkmale der Erfindung ergeben sich durch die nachfolgende Beschreibung von bevorzugten Ausführungsformen in Form von Beispielen in Verbindung mit den Unteransprüchen. Hierbei können einzelne Merkmale der Erfindung alleine oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### Beispiele

**Tabelle 1:**

| Konzentration | Viskosität | Viskosität |
|---|---|---|
| Komponente c (PVA) | [mPas] (bei 20 °C) | [mPas] (bei 37 °C) |
| und b+c (FeCl₃+PVA) | | |
| 5 Gew.% PVA | 120 | 60 |
| 7,5 Gew.% PVA | 750 | 450 |
| 10 Gew.% PVA | 3050 | 1600 |
| 11,5 Gew.% PVA | 4000 | 3100 |
| 5 Gew.% PVA / | 90 | 60 |
| 1 Gew.% FeCl₃ | | |
| 7,5Gew.% PVA / | 750 | 400 |
| 1 Gew.% FeCl₃ | | |
| 10Gew.% PVA / | 1700 | 1500 |
| 1 Gew.% FeCl₃ | | |
| 11,5Gew.% PVA / | 4800 | 3200 |
| 1 Gew.% FeCl₃ | | |

**Tabelle 2: Viskositäten der Komponente (a) (CMC)**

| Konzentration | Viskosität |
|---|---|
| Komponente a (CMC) | [mPas] (bei 37 °C) |
| 1 Gew.% | 180 |
| 1,5 Gew.% | 400 |
| 2 Gew.% | 1500 |
| 2,25 Gew.% | nicht linear |
| 2,4 Gew.% | nicht linear |
| 2.5 Gew.% | nicht linear |

**Tabelle 3: Viskositäten der Komponenten (a) (CMC), (c) (PVA), (b/c) (FeCl₃/PVA) und (a + b/c) (CMC+ FeCl₃/PVA) und des Gelierungsproduktes**

| Konzentrationen | Viskosität [mPas] (bei 37 °C) |
|---|---|
| 2,5 Gew.% CMC | 1402 |
| 3 Gew.% CMC | 2423 |
| 10 Gew.% PVA | 1423 |
| 10 Gew.% PVA / 0,5 Gew.% FeCl₃ | 1700 |
| 10 Gew.% PVA / 1 Gew.% FeCl₃ | 1499 |
| 11 Gew.% PVA | 2534 |
| 11 Gew.% PVA / 0,5 Gew.% FeCl₃ | 2846 |
| 11 Gew.% PVA / 1 Gew.% FeCl₃ | 2451 |
| 2,5 Gew.% CMC + | 277883 |
| (10 Gew.% PVA / 0,5 Gew.% FeCl₃) | |
| 2,5 Gew.% CMC + | 504851 |
| (10 Gew.% PVA / 1 Gew.% FeCl₃) | |
| 3 Gew.% CMC + | 384091 |
| (11 Gew.% PVA / 0,5 Gew.% FeCl₃) | |
| 3 Gew.% CMC + | 584084 |
| (11 Gew.% PVA / 1 Gew.% FeCl₃) | |

### Beispiele für Komponente (a) mit Verdickungsmitteln

| | |
|---|---|
| Alginat: | 3 Gew.% CMC : 5 Gew.% Alginat (1:1 Volumenverhältnis) |
| | Viskosität 4,39 Pas bei 37 °C |
| Xanthan: | 3 Gew.% CMC : 3 Gew.% Xanthan (1:1 Volumenverhältnis) |
| | Viskosität 2,38 Pas bei 37 °C |

### Beispiele der erfindungsgemäßen Zusammensetzung umfassend Komponente a, b und c:

### a) Verstreichbare Ausführungsform der Zusammensetzung

Die Komponenten CMC und (PVA/FeCl₃) werden jeweils im Volumenverhältnis 1:1 eingesetzt.
0,5 Gew.% CMC + (15 Gew.% PVA / 0,1 Gew.% FeCl₃)
(Endkonzentrationen in der Zusammensetzung: 0,25 Gew.% CMC / 7,5 Gew.% PVA / 0,05 Gew.% FeCl₃)
0,5 Gew.% CMC + (15 Gew.% PVA / 2 Gew.% FeCl₃)

1 Gew.% CMC + (15 Gew.% PVA / 0,1 Gew.% FeCl₃)
1 Gew.% CMC + (15 Gew.% PVA / 0,5 Gew.% FeCl₃)
1 Gew.% CMC + (15 Gew.% PVA / 1 Gew.% FeCl₃)

2 Gew.% CMC + (10 Gew.% PVA / 0,5 Gew.% FeCl₃)
2 Gew.% CMC + (10 Gew.% PVA / 1 Gew.% FeCl₃)
2 Gew.% CMC + (10 Gew.% PVA / 2 Gew.% FeCl₃)

2,5 Gew.% CMC + (10 Gew.% PVA / 0,5 Gew.% FeCl₃)
2,5 Gew.% CMC + (10 Gew.% PVA / 1 Gew.% FeCl₃)
2,5 Gew.% CMC + (10 Gew.% PVA / 2 Gew.% FeCl₃)

3 Gew.% CMC + (10 Gew.% PVA / 0,1 Gew.% FeCl₃)
3 Gew.% CMC + (10 Gew.% PVA / 0,5 Gew.% FeCl₃)
3 Gew.% CMC + (10 Gew.% PVA / 1 Gew.% FeCl₃)

3 Gew.% CMC + (15 Gew.% PVA / 0,1 Gew.% FeCl₃)
3 Gew.% CMC + (15 Gew.% PVA / 0,5 Gew.% FeCl₃)
3 Gew.% CMC + (15 Gew.% PVA / 1 Gew.% FeCl₃)

4 Gew.% CMC + (10 Gew.% PVA / 0,5 Gew.% FeCl₃)
4 Gew.% CMC + (10 Gew.% PVA / 1 Gew.% FeCl₃)
4 Gew.% CMC + (10 Gew.% PVA / 2 Gew.% FeCl₃)
5 Gew.% CMC + (10 Gew.% PVA / 1 Gew.% FeCl₃)
5 Gew.% CMC + (10 Gew.% PVA / 2 Gew.% FeCl₃)

### b) Versprühbare Ausführungsform der Zusammensetzung

1 Gew.% CMC + (10 Gew.% PVA / 0,1 Gew.% FeCl₃)
1 Gew.% CMC + (10 Gew.% PVA / 0,5 Gew.% FeCl₃)
1 Gew.% CMC + (10 Gew.% PVA / 1 Gew.% FeCl₃)

2 Gew.% CMC + (10 Gew.% PVA / 0,5 Gew.% FeCl₃)
2 Gew.% CMC + (10 Gew.% PVA / 1 Gew.% FeCl₃)
2 Gew.% CMC + (10 Gew.% PVA / 2 Gew.% FeCl₃)

2,5 Gew.% CMC + (10 Gew.% PVA / 0,5 Gew.% FeCl₃)
2,5 Gew.% CMC + (10 Gew.% PVA / 1 Gew.% FeCl₃)
2,5 Gew.% CMC + (10 Gew.% PVA / 2 Gew.% FeCl₃)

3 Gew.% CMC + (10 Gew.% PVA / 0,1 Gew.% FeCl₃)
(Endkonzentrationen in der Zusammensetzung: 1,5 Gew.% CMC / 5
Gew.% PVA / 0,05 Gew.% FeCl₃)
3 Gew.% CMC + (10 Gew.% PVA / 0,5 Gew.% FeCl₃)
3 Gew.% CMC + (10 Gew.% PVA / 1 Gew.% FeCl₃)

3 Gew.% CMC + (15 Gew.% PVA / 0,1 Gew.% FeCl₃)
3 Gew.% CMC + (15 Gew.% PVA / 0,5 Gew.% FeCl₃)
3 Gew.% CMC + (15 Gew.% PVA / 1 Gew.% FeCl₃)

4 Gew.% CMC + (10 Gew.% PVA / 0,5 Gew.% FeCl₃)
4 Gew.% CMC + (10 Gew.% PVA / 1 Gew.% FeCl₃)
4 Gew.% CMC + (10 Gew.% PVA / 2 Gew.% FeCl₃)

5 Gew.% CMC + (10 Gew.% PVA / 1 Gew.% FeCl₃)
5 Gew.% CMC + (10 Gew.% PVA / 2 Gew.% FeCl₃)

### Beispiele zur Vernetzung der Komponente (c) (PVA) und zur Herstellung eines bereits vernetzten Gels

a) Ein Gel mit der Gesamt-Zusammensetzung von 10 Gew.% PVA, 3 Gew.% Zitronensäure und 1 Gew.% Carboxymethylcellulose geliert und weist eine Viskosität von 7,8 ± 0,2 Pas auf. Das Gel wurde mittels Dampf sterilisiert und mit 3 Gew.% Natriumhydrogencarbonat auf einen pH-Wert von 5,4 eingestellt.
   Das Gel lässt sich gut auf feuchtem Gewebe applizieren und haftet im rabbit side wall Modell gut am Gewebe. Die Verträglichkeit im Tier ist sehr gut, die postoperative Beobachtung zeigte kein verändertes Verhalten. Nach der Sektion ist das in der OP verletzte Gewebe mit Granulationsgewebe überzogen und frei von Verwachsungen.
   Die Zerfallszeit des Gels wurde in einem Tablettenzerfallszeittester der Firma Ischi durchgeführt. Die mittleren Zerfallszeiten des Gels betrug 10 ± 2 Minuten. Im Vergleich dazu eine Mischung aus PVA und CMC mit gleichen Materialeinwaagen zerfallen innerhalb 1 Minute. Durch die erhaltene Gelierung kann also auch die Resorptionszeit des Gels in vivo eingestellt werden.
b) Ein Gel mit der Gesamtzusammensetzung von 20 Gew.% PVA, 1 Gew.% Carboxymethylcellulose und 10 Gew.% Silberzitrat geliert und weist eine Viskosität von 3,9 ± 0,2 Pas auf. Mittels einer Zugabe von Natriumhydrogencarbonat kann ein physiologisch verträglicher pH-Wert von 5 eingestellt werden. Die Silberionen werden in vivo freigesetzt und zeigen eine antibakterielle Wirkung. Die Wirksamkeit von Silberzitrat auf S. aureus, E. coli und P. aeroginosa wurde in Tests bewiesen.

## Patentansprüche

1. Zusammensetzung zur Adhäsionsprophylaxe für die postoperative Verhinderung von Verwachsungen im Körper aus mindestens drei, insbesondere drei, Komponenten, wobei mindestens zwei Komponenten derart miteinander vernetzbar sind, dass die Zusammensetzung nach der Vernetzung eine homogene gelartige Masse ist, umfassend mindestens folgende Komponenten:
a) wässrige, insbesondere viskose, Lösung einer Carboxymethylcellulose (CMC),
b) wässrige Lösung mindestens eines Vernetzungsmittels, insbesondere eines Gelbildners,
c) wässrige Lösung eines Polyvinylalkohols (PVA).

2. Zusammensetzung nach Anspruch 1, **dadurch gekennzeichnet, dass** Komponente a) und/oder Komponente c) mit der Komponente b) vernetzbar sind.

3. Zusammensetzung nach Anspruch 1 oder 2, **dadurch gekennzeichnet, dass** die Vernetzungszeit der Zusammensetzung, insbesondere durch Art und Mengenverhältnis der mindestens zwei miteinander vernetzbaren Komponenten untereinander, je nach Verwendung, einstellbar ist.

4. Zusammensetzung nach einem der vorhergehenden Ansprüche, **dadurch gekennzeichnet, dass** die Komponenten und deren Verhältnis so ausgewählt sind, dass die Vernetzungszeit der Zusammensetzung 5 Sekunden bis 10 Minuten, vorzugsweise 10 Sekunden bis 5 Minuten, insbesondere 30 bis 60 Sekunden, beträgt.

5. Zusammensetzung nach einem der vorhergehenden Ansprüche, **dadurch gekennzeichnet, dass** die Vernetzungszeit der Zusammensetzung durch die Konzentrationen der vernetzbaren Komponenten und/oder durch den Grad der Vermischung der einzelnen Komponenten einstellbar ist.

6. Zusammensetzung nach einem der vorhergehenden Ansprüche, **dadurch gekennzeichnet, dass** jede Komponente vor der Vernetzung separat applizierbar, vorzugsweise verstreichbar oder versprühbar, ist.

7. Zusammensetzung nach einem der vorhergehenden Ansprüche, **dadurch gekennzeichnet, dass** die Zusammensetzung mindestens ein Mittel zur Erhöhung der Viskosität, vorzugsweise ein Verdickungsmittel oder ein Geliermittel, insbesondere aus der Gruppe bestehend aus Xanthan, Pullulan, Curdlan, Alginate, Agar-Agar, Carrageen, Furcelleran, Pektin, Johannesbrotkernmehl, Guarkernmehl, Tarakernmehl, Gummiarabikum, Traganth, Karaya, Cellulose und Stärke, enthält.

8. Zusammensetzung nach einem der vorhergehenden Ansprüche, **dadurch gekennzeichnet, dass** Komponente (a) 0,1 bis 5 Gew%, vorzugsweise 2 bis 4 Gew%, insbesondere ca. 3 Gew%, CMC enthält.

9. Zusammensetzung nach einem der vorhergehenden Ansprüche, **dadurch gekennzeichnet, dass** Komponente (a) eine Viskosität von 0,5 Pas bis 5 Pas, insbesondere von 2,5 Pas bei einer 3 Gew.% CMC-Lösung, aufweist.

10. Zusammensetzung nach einem der vorhergehenden Ansprüche, **dadurch gekennzeichnet, dass** Komponente (c) 1 bis 20 Gew%, vorzugsweise 5 bis 15 Gew%, insbesondere ca. 10 Gew%, PVA enthält.

11. Zusammensetzung nach einem der vorhergehenden Ansprüche, **dadurch gekennzeichnet, dass** Komponente (c) (PVA) eine Viskosität von 0,1 Pas bis 50 Pas, vorzugsweise 0,5 Pas bis 20 Pas, insbesondere ca. 1,4 Pas bei einer 10 Gew% PVA Lösung, aufweist.

12. Zusammensetzung nach einem der vorhergehenden Ansprüche, **dadurch gekennzeichnet, dass** der PVA ein Molekulargewicht von 20.000 bis 400.000, vorzugsweise 100.000 bis 300.000, insbesondere ca. 200.000, aufweist.

13. Zusammensetzung nach einem der vorhergehenden Ansprüche, **dadurch gekennzeichnet, dass** das Volumenverhältnis von Komponente (a) zu Komponente (b/c) 1:9 bis 9:1, vorzugsweise 1:1, ist.

14. Zusammensetzung nach einem der vorhergehenden Ansprüche, **dadurch gekennzeichnet, dass** Komponente (b) 0,001 - 2 Gew%, vorzugsweise 0,25 - 0,5 Gew%, insbesondere 0,1 - 0,5 Gew%, Vernetzungsmittel enthält.

15. Zusammensetzung nach einem der vorhergehenden Ansprüche, **dadurch gekennzeichnet, dass** es sich bei dem Vernetzungsmittel der Komponente (b) mit Komponente (a) um eine Lösung mehrwertiger Ionen, insbesondere mehrwertiger Metallionen, vorzugsweise Aluminium-, Eisen(III)- und/oder Kupfer(II)ionen und insbesondere bevorzugt um Eisen(III)chlorid (FeCl₃), handelt.

16. Zusammensetzung nach einem der vorhergehenden Ansprüche, **dadurch gekennzeichnet, dass** es sich bei dem Vernetzungsmittel der Komponente (b) mit Komponente (a) um eine Lösung mehrwertiger organischer Kationen, vorzugsweise Polyaminosäuren, insbesondere Poly-Lysine, handelt.

17. Zusammensetzung nach einem der vorhergehenden Ansprüche, **dadurch gekennzeichnet, dass** es sich bei dem Vernetzungsmittel der Komponente (b) mit Komponente (c) um eine wässrige Lösung einer mehrwertigen organischen Säure, insbesondere von Zitronensäure und/oder einer ihrer löslichen Salze, handelt.

18. Zusammensetzung nach Anspruch 17, **dadurch gekennzeichnet, dass** es sich bei dem Salz der mehrwertigen organischen Säure um ein Silbersalz handelt.

19. Zusammensetzung nach einem der vorhergehenden Ansprüche, **dadurch gekennzeichnet, dass** die Vernetzung der Komponenten durch Komplexbildung, vorzugsweise durch koordinative Bindungen wie ionische, van der Waal oder H-Brücken Bindungen, erfolgt.

20. Zusammensetzung nach einem der vorhergehenden Ansprüche, **dadurch gekennzeichnet, dass** sie einen Wassergehalt von mindestens 70 %, vorzugsweise mindestens 80 %, insbesondere 80 % bis 90 % aufweist.

21. Zusammensetzung nach einem der vorhergehenden Ansprüche, **dadurch gekennzeichnet, dass** sie einen antibakteriellen Zusatz enthält.

22. Zusammensetzung nach einem der vorhergehenden Ansprüche, **dadurch gekennzeichnet, dass** sie in vernetztem Zustand flexibel ist.

23. Zusammensetzung nach einem der vorhergehenden Ansprüche, **dadurch gekennzeichnet, dass** sie in bereits vernetztem Zustand zur Applikation vorgesehen sind und die Viskosität des vernetzten Gels bei 3 bis 50 Pas, insbesondere 3 bis 20 Pas, liegt.

24. Kit, enthaltend mindestens zwei, vorzugsweise zwei, getrennte Behältnisse, wobei die Behältnisse mindestens eine der Komponenten (a),(b) und (c) der Zusammensetzung nach einem der Ansprüche 1 bis 22 enthalten.

25. Kit nach Anspruch 24, **dadurch gekennzeichnet, dass** er eine Vorrichtung zum Mischen der Komponenten, insbesondere einen statischen oder dynamischen Mischer, aufweist.

26. Kit nach einem der Ansprüche 24 oder 25, **dadurch gekennzeichnet, dass** er für den minimal invasiven, insbesondere laparoskopischen, chirurgischen Einsatz ausgelegt ist.

27. Bereitstellung der Zusammensetzung nach einem der Ansprüche 1 bis 23 zur Verwendung zur Adhäsionsprophylaxe für die postoperative Verhinderung von Verwachsungen im Körper.

28. Bereitstellung der Zusammensetzung nach einem der Ansprüche 1 bis 23 zur Verwendung zum Auffüllen von AVMs (arteriovacuösen malformations) und/oder Aneurismen.

## Claims

1. Composition for adhesion prophylaxis for the post-operative prevention of adhesions in the body comprising at least three, in particular three, components, where at least two components are crosslinkable with one another such that the composition after crosslinking is a homogeneous gel-like mass, comprising at least the following components:
a) aqueous, in particular viscous, solution of a carboxymethylcellulose (CMC),
b) aqueous solution of at least one crosslinking agent, in particular of a gel former,
c) aqueous solution of a polyvinyl alcohol (PVA).

2. Composition according to Claim 1, **characterized in that** component a) and/or component c) are crosslinkable with component b).

3. Composition according to Claim 1 or 2, **characterized in that** the crosslinking time of the composition can be adjusted in particular by means of the type and quantitative ratio of the at least two mutually crosslinkable components with one another, according to use.

4. Composition according to one of the preceding claims, **characterized in that** the components and the ratio thereof are selected such that the crosslinking time of the composition is 5 seconds to 10 minutes, preferably 10 seconds to 5 minutes, in particular 30 to 60 seconds.

5. Composition according to one of the preceding claims, **characterized in that** the crosslinking time of the composition can be adjusted by means of the concentrations of the crosslinkable components and/or by means of the degree of mixing of the individual components.

6. Composition according to one of the preceding claims, **characterized in that** each component prior to the crosslinking can be applied separately, and is preferably spreadable or sprayable.

7. Composition according to one of the preceding claims, **characterized in that** the composition comprises at least one agent for increasing the viscosity, preferably a thickener or a gelling agent, in particular from the group consisting of xanthan, pullulan, curdlan, alginates, agar agar, carrageen, furcelleran, pectin, carob seed flour, guar seed flour, tara seed flour, gum arabic, tragacanth, karaya, cellulose and starch.

8. Composition according to one of the preceding claims, **characterized in that** component (a) comprises 0.1 to 5% by weight, preferably 2 to 4% by weight, in particular ca. 3% by weight, of CMC.

9. Composition according to one of the preceding claims, **characterized in that** component (a) has a viscosity of from 0.5 Pas to 5 Pas, in particular from 2.5 Pas for a 3% by weight CMC solution.

10. Composition according to one of the preceding claims, **characterized in that** component (c) comprises 1 to 20% by weight, preferably 5 to 15% by weight, in particular ca. 10% by weight, of PVA.

11. Composition according to one of the preceding claims, **characterized in that** component (c) (PVA) has a viscosity of from 0.1 Pas to 50 Pas, preferably 0.5 Pas to 20 Pas, in particular ca. 1.4 Pas for a 10% by weight PVA solution.

12. Composition according to one of the preceding claims, **characterized in that** the PVA has a molecular weight of from 20 000 to 400 000, preferably 100 000 to 300 000, in particular ca. 200 000.

13. Composition according to one of the preceding claims, **characterized in that** the volume ratio of component (a) to component (b/c) is 1:9 to 9:1, preferably 1:1.

14. Composition according to one of the preceding claims, **characterized in that** component (b) comprises 0.001-2% by weight, preferably 0.25-0.5% by weight, in particular 0.1-0.5% by weight, of crosslinking agent.

15. Composition according to one of the preceding claims, **characterized in that** the crosslinking agent of component (b) with component (a) is a solution of polyvalent ions, in particular polyvalent metal ions, preferably aluminium, iron(III) and/or copper(II) ions, and particularly preferably iron(III) chloride (FeCl₃).

16. Composition according to one of the preceding claims, **characterized in that** the crosslinking agent of component (b) with component (a) is a solution of polyvalent organic cations, preferably polyamino acids, in particular polylysines.

17. Composition according to one of the preceding claims, **characterized in that** the crosslinking agent of component (b) with component (c) is an aqueous solution of a polyvalent organic acid, in particular of citric acid and/or one of its soluble salts.

18. Composition according to Claim 17, **characterized in that** the salt of the polyhydric organic acid is a silver salt.

19. Composition according to one of the preceding claims, **characterized in that** the crosslinking of the components takes place by means of complex formation, preferably by means of coordinative bonds such as ionic, van der Waal or H-bridge bonds.

20. Composition according to one of the preceding claims, **characterized in that** it has a water content of at least 70%, preferably at least 80%, in particular 80% to 90%.

21. Composition according to one of the preceding claims, **characterized in that** it comprises an antibacterial additive.

22. Composition according to one of the preceding claims, **characterized in that** it is flexible in the crosslinked state.

23. Composition according to one of the preceding claims, **characterized in that** it is intended for application in the ready-crosslinked state and the viscosity of the crosslinked gel is 3 to 50 Pas, in particular 3 to 20 Pas.

24. Kit containing at least two, preferably two, separate containers, where the containers comprise at least one of the components (a), (b) and (c) of the composition according to one of Claims 1 to 22.

25. Kit according to Claim 24, **characterized in that** it has a device for mixing the components, in particular a static or dynamic mixer.

26. Kit according to one of Claims 24 or 25, **characterized in that** it is designed for minimally invasive, in particular laparoscopic, surgical intervention.

27. Provision of the composition according to one of Claims 1 to 23 for use for adhesion prophylaxis for the post-operative prevention of adhesions in the body.

28. Provision of the composition according to one of Claims 1 to 23 for use for filling AVMs (arteriovenous malformations) and/or aneurysms.

## Revendications

1. Composition destinée à la prophylaxie des adhérences pour empêcher les adhérences post-opératoires dans le corps, constituée par au moins trois, en particulier trois, composants, au moins deux composants étant réticulables les uns avec les autres de manière telle que la composition est une masse de type gel homogène après la réticulation, comprenant au moins les composants suivants :
a) une solution aqueuse, en particulier visqueuse d'une carboxyméthylcellulose (CMC),
b) une solution aqueuse d'au moins un réticulant, en particulier d'un gélifiant,
c) une solution aqueuse d'un poly(alcool vinylique) (PVA).

2. Composition selon la revendication 1, **caractérisée en ce que** le composant a) et/ou le composant c) sont réticulables avec le composant b).

3. Composition selon la revendication 1 ou 2, **caractérisée en ce que** le temps de réticulation de la composition est réglable, en particulier par le type et le rapport des quantités desdits au moins deux composants réticulables l'un avec l'autre, en fonction de l'utilisation.

4. Composition selon l'une quelconque des revendications précédentes, **caractérisée en ce que** les composants et leur rapport sont choisis de manière telle que le temps de réticulation de la composition est de 5 secondes à 10 minutes, de préférence de 10 secondes à 5 minutes, en particulier de 30 à 60 secondes.

5. Composition selon l'une quelconque des revendications précédentes, **caractérisée en ce que** le temps de réticulation de la composition est réglable par les concentrations en composants réticulables et/ou par le degré de mélange des différents composants.

6. Composition selon l'une quelconque des revendications précédentes, **caractérisée en ce que** chaque composant est applicable séparément, en pouvant de préférence être enduit ou pulvérisé, avant la réticulation.

7. Composition selon l'une quelconque des revendications précédentes, **caractérisée en ce que** la composition contient au moins un agent pour augmenter la viscosité, de préférence un épaississant ou un gélifiant, en particulier du groupe formé par le xanthane, le pullulan, le curdlan, les alginates, l'agar-agar, le carragheen, le furcelleran, la pectine, la farine de graines de caroube, la gomme Guar, la gomme Tara, la gomme arabique, la gomme adragante, la gomme Karaya, la cellulose et l'amidon.

8. Composition selon l'une quelconque des revendications précédentes, **caractérisée en ce que** le composant (a) contient 0,1 à 5% en poids, de préférence 2 à 4% en poids, en particulier environ 3% en poids, de CMC.

9. Composition selon l'une quelconque des revendications précédentes, **caractérisée en ce que** le composant (a) présente une viscosité de 0,5 Pa.s à 5 Pa.s, en particulier de 2,5 Pa.s, mesurée sur une solution à 3% en poids de CMC.

10. Composition selon l'une quelconque des revendications précédentes, **caractérisée en ce que** le composant (c) contient 1 à 20% en poids, de préférence 5 à 15% en poids, en particulier environ 10% en poids, de PVA.

11. Composition selon l'une quelconque des revendications précédentes, **caractérisée en ce que** le composant (c) (PVA) présente une viscosité de 0,1 Pa.s à 50 Pa.s, de préférence de 0,5 Pa.s à 20 Pa.s, en particulier d'environ 1,4 Pa.s, mesurée sur une solution de 10% en poids de PVA.

12. Composition selon l'une quelconque des revendications précédentes, **caractérisée en ce que** le PVA présente un poids moléculaire de 20 000 à 400 000, de préférence de 100 000 à 300 000, en particulier d'environ 200 000.

13. Composition selon l'une quelconque des revendications précédentes, **caractérisée en ce que** le rapport volumique du composant (a) au composant (b/c) est de 1:9 à 9:1, de préférence de 1:1.

14. Composition selon l'une quelconque des revendications précédentes, **caractérisée en ce que** le composant (b) contient 0,001-2% en poids, de préférence 0,25-0,5% en poids, en particulier 0,1-0,5% en poids, de réticulant.

15. Composition selon l'une quelconque des revendications précédentes, **caractérisée en ce qu'**il s'agit, pour le réticulant du composant (b) avec le composant (a), d'une solution d'ions polyvalents, en particulier d'ions métalliques polyvalents, de préférence d'ions d'aluminium, de fer (III) et/ou de cuivre (II) et de manière particulièrement préférée de chlorure de fer (III) (FeCl₃) .

16. Composition selon l'une quelconque des revendications précédentes, **caractérisée en ce qu'**il s'agit, pour le réticulant du composant (b) avec le composant (a), d'une solution de cations organiques polyvalents, de préférence de poly(acides aminés), en particulier de polylysine.

17. Composition selon l'une quelconque des revendications précédentes, **caractérisée en ce qu'**il s'agit, pour le réticulant du composant (b) avec le composant (c), d'une solution aqueuse d'acides organiques polyvalents, de préférence d'acide citrique et/ou d'un de ses sels solubles.

18. Composition selon la revendication 17, **caractérisée en ce qu'**il s'agit, pour le sel de l'acide organique polyvalent, d'un sel d'argent.

19. Composition selon l'une quelconque des revendications précédentes, **caractérisée en ce que** la réticulation des composants a lieu par complexation, de préférence par des liaisons de coordination, telles que des liaisons ioniques, de van der Waals ou des ponts d'hydrogène.

20. Composition selon l'une quelconque des revendications précédentes, **caractérisée en ce qu'**elle présente une teneur en eau d'au moins 70%, de préférence d'au moins 80%, en particulier de 80% à 90%.

21. Composition selon l'une quelconque des revendications précédentes, **caractérisée en ce qu'**elle contient un additif antibactérien.

22. Composition selon l'une quelconque des revendications précédentes, **caractérisée en ce qu'**elle est souple à l'état réticulé.

23. Composition selon l'une quelconque des revendications précédentes, **caractérisée en ce qu'**elle est prévue pour l'application dans un état déjà réticulé et la viscosité du gel réticulé se situe à 3 jusqu'à 50 Pa.s, de préférence à 3 jusqu'à 20 Pa.s.

24. Kit, contenant au moins deux, de préférence deux récipients séparés, où les récipients contiennent au moins un des composants (a), (b) et (c) de la composition selon l'une quelconque des revendications 1 à 22.

25. Kit selon la revendication 24, **caractérisé en ce qu'**il présente un dispositif pour le mélange des composants, en particulier un mélangeur statique ou dynamique.

26. Kit selon l'une quelconque des revendications 24 ou 25, **caractérisé en ce qu'**il est conçu pour une utilisation chirurgicale invasive minimale, en particulier laparoscopique.

27. Préparation de la composition selon l'une quelconque des revendications 1 à 23 destinée à une utilisation pour la prophylaxie des adhérences pour empêcher les adhérences post-opératoires dans le corps.

28. Préparation de la composition selon l'une quelconque des revendications 1 à 23 destinée à une utilisation pour le remplissage d'AVM (malformations artérioveineuses) et/ou aneurismes.
